# EUROPEAN PATENT SPECIFICATION

(11) **EP 2 059 199 B1**
(45) Date of publication and mention of the grant of the patent: **01.08.2012**
(21) Application number: 07809466.1
(22) Date of filing: 12.06.2007
(51) Int. Cl.: A61F 2/90

(54) **LONGITUDINALLY FLEXIBLE EXPANDABLE STENT**
IN LÄNGSRICHTUNG FLEXIBLER EXPANDIERBARER STENT
ENDOPROTHÈSE VASCULAIRE EXTENSIBLE ET LONGITUDINALEMENT FLEXIBLE

(30) Priority: 12.09.2006 US 519552
(43) Date of publication of application: 20.05.2009
(73) Proprietor: Boston Scientific Limited, Christ Church (BB)
(72) Inventor: BROWN, Brian J., Hanover, MN 55341 (US); DAVIS, Michael L., Rockford, MN 55373 (US); MEYER, Michael P., Richfield, Minnesota 55423 (US); GREGORICH, Daniel, St. Louis Park, 55416 (US); CHOUINARD, Paul F., Maple Grove, MN 55311 (US)
(74) Representative: Hauck Patent- und Rechtsanwälte
(86) International application number: PCT/US2007/013714
(87) International publication number: WO 2008/033174

(56) References cited:
- EP-A- 1 025 812
- WO-A-01/01889
- WO-A-2007/053224
- US-A1- 2003 225 448

## Description

### BACKGROUND OF THE INVENTION

### Field of the Invention

In some embodiments this invention relates to implantable medical devices, their manufacture.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminal and enlarged radially after being introduced percutaneously. Stents may be implanted in a variety of body lumens or vessels such as within the vascular system, urinary tracts, bile ducts, fallopian tubes, coronary vessels, secondary vessels, etc. Stents may be used to reinforce body vessels and to prevent restenosis following angioplasty in the vascular system. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braid.

WO 01/01889 A describes a self-expanding tubular stent comprising a plurality of stent segments. Each stent segment is formed of an elongate ribbon having portions cut therefrom to form a wave-like undulating pattern, opposed edges of which are attached to one another so as to form a generally cylindrical configuration. A disclosed method effects formation of such an expandable tubular stent by fast providing an elongate flat ribbon of biocompatible stent material and selectively removing portions of such material to form an undulating wave-like pattern along the length of the ribbon. The ribbon is then coiled into a generally cylindrical configuration and opposed ends of the ribbon are secured to one another to form a generally cylindrical and expandable spiral stent section.

### BRIEF SUMMARY OF THE INVENTION

In some embodiments, the invention provides a tubular expandable stent as disclosed in the appended claims, comprising a plurality of cylindrical shaped open cylindrical segments aligned on a common longitudinal axis to define a generally tubular stent body, each segment being defined by a member formed in an undulating flexible pattern of interconnected substantially parallel struts with pairs thereof having alternating interconnecting end portions to define the periphery of the expandable stent segment, and in which the connected end portions of paired struts in each segment, before the stent is expanded, are positioned substantially opposite to connected end portions of paired struts in adjacent segments. The segments are interconnected by a plurality of interconnecting elements extending from some of the connected end portions on one segment to some of the connected end portions on adjacent segments in such a manner that there are three or more legs between points of connection from one side of each segment to its other side. Additionally, the connecting elements extend angularly from connecting end portion of one segment to connecting end portion of an adjacent segment, not to an opposite connecting end portion on an adjacent segment whereby upon expansion of the stent the adjacent segments are displaced relative to each other about the periphery of the stent body to accommodate flexing of the stent within paired struts without interference between adjacent segments, rather than by means of articulating flexible connectors between segments. As a result, the connectors between the segments are not intended to flex or bend under normal use.

In at least one embodiment, a stent comprises a plurality of serpentine bands and a plurality of connector struts. Adjacent serpentine bands are connected by at least one connector strut. Each serpentine band comprises a plurality of alternating straight band struts and turns. Each connector strut is connected at one end to a turn of one serpentine band and connected at the other end to a turn of another serpentine band. The turns of a serpentine band comprise connected turns that connect to a connector strut and unconnected turns that do not connect to a connector strut. At least one of the serpentine bands comprises a repeating pattern of three band struts and then five band struts extending between connected turns as the serpentine band is traversed.

In at least one embodiment, a stent comprises a plurality of serpentine bands and a plurality of connector columns. Each serpentine band comprises straight band struts extending between turns. The turns comprise alternating proximal peaks and distal valleys. Each connector column comprises a plurality of connector struts, each connector strut connecting between a proximal peak of one serpentine band and a distal valley of another serpentine band. At least one serpentine band comprises three band struts extending between a first connected proximal peak that connects to a connector strut and a first connected distal valley that connects to a connector strut. The serpentine band further comprises five band struts extending between the first connected distal valley and a second connected proximal peak.

In at least one embodiment, a stent comprises a plurality of serpentine bands and a plurality of connector columns. Each serpentine band comprises a plurality of alternating straight band struts and turns. Adjacent serpentine bands are connected across a connector column by a plurality of connector struts. Each connector strut is connected at one end to a turn of one serpentine band and connected at the other end to a turn of another serpentine band. The turns of a serpentine band comprise connected turns that connect to a connector strut and unconnected turns that do not connect to a connector strut. At least one of the serpentine bands comprises two unconnected turns between a first connected turn and a second connected turn and four unconnected turns between the second connected turn and a third connected turn as the serpentine band is traversed.

In at least one embodiment, a stent comprises a plurality of serpentine bands including a first serpentine band, a second serpentine band and a third serpentine band, and a plurality of connector struts including a first connector strut, a second connector strut and a third connector strut. Each serpentine band comprises alternating proximal peaks and distal valleys connected by straight band struts. The proximal peaks include connected proximal peaks that connect to a connector strut and unconnected proximal peaks that do not connect to a connector strut. The distal valleys include connected distal valleys that connect to a connector strut and unconnected distal valleys that do not connect to a connector strut. Each connector strut connects between a connected distal valley of one serpentine band and a connected proximal peak of another serpentine band. The first connector strut connects between a first connected distal valley of the first serpentine band and a portion of the second serpentine band. The second connector strut connects between the second serpentine band and the third serpentine band. The third connector strut connects to a second connected distal valley of the third serpentine band. Proximal peaks of the first serpentine band are circumferentially offset from proximal peaks of the second serpentine band. Proximal peaks of the first serpentine band are circumferentially aligned with proximal peaks of the third serpentine band. The first connected distal valley is circumferentially aligned with a first unconnected distal valley of the third serpentine band. The first unconnected distal valley is circumferentially adjacent to the second connected distal valley.

These and other embodiments which characterize the invention are pointed out with particularity in the claims annexed hereto and forming a part hereof. However, for further understanding of the invention, its advantages and objectives obtained by its use, reference should be made to the drawings which form a further part hereof and the accompanying descriptive matter, in which there are illustrated and described further embodiments of the invention.

### BRIEF DESCRIPTION OF THE SEVERAL VIEWS OF THE DRAWING(S)

A detailed description of the invention is hereafter described with specific reference being made to the drawings.
Figure 1 shows a flat view of an example of an unexpanded stent configuration. Figure 2 shows the pattern of Figure 1 in a tubular, unexpanded stent.
Figure 3 shows an expanded stent of the stent shown in Figure 1.
Figure 4 shows a flat view of an alternate unexpanded stent.
Figure 5 shows a flat pattern for an embodiment of a stent according to the invention.
Figure 6 shows a three-dimensional isometric view of an embodiment of a stent.
Figure 7 shows a flat pattern depiction of the stent pattern of Figure 5 in a state of expansion that is greater than that depicted in Figure 5.
Figure 8 shows a flat pattern depiction of the stent pattern of Figure 5 in a state of expansion that is greater than that depicted in Figure 7.
Figure 9 shows a flat pattern depiction of the stent pattern of Figure 5 in a state of expansion that is greater than that depicted in Figure 8.
Figure 10 shows a flat pattern depiction of the stent pattern of Figure 5 in a state of expansion that is greater than that depicted in Figure 9. The state of expansion shown can be considered a state of overexpansion.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

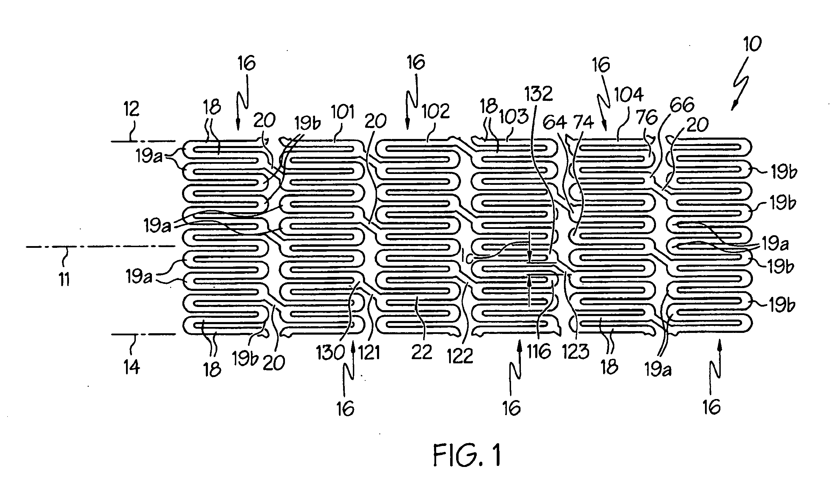

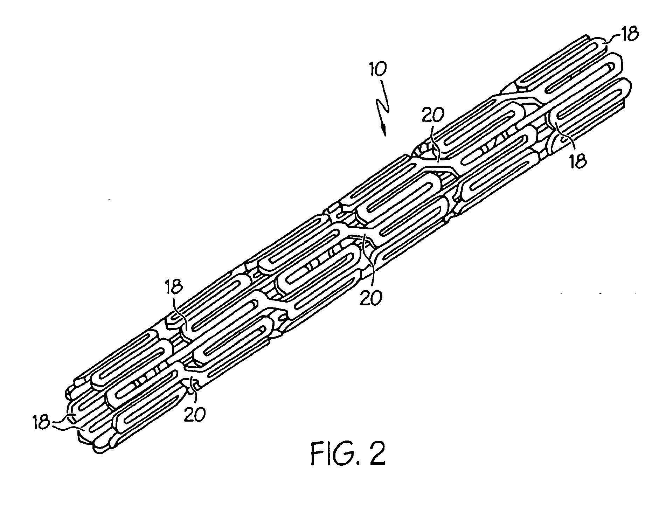

Turning to the Figures, Figure 1 and Figure 2 show a fragmentary flat view of an unexpanded stent configuration and the actual tubular stent (unexpanded), respectively. That is, the stent is shown for clarity in Figure 1 in the flat and may be made from a flat pattern 10 (Figure 1) which is formed into a tubular shape by rolling the pattern so as to bring edges 12 and 14 together (Figure 1). The edges may then joined as by welding or the like to provide a configuration such as that shown in Figure 2.

The configuration can be seen in these Figures to be made up of a plurality of adjacent segments generally indicated at 16, each of which is formed in an undulating flexible pattern of substantially parallel struts 18. Pairs of struts are interconnected at alternating end portions 19a and 19b. As is seen in Figure 1, the interconnecting end portions 19b of one segment are positioned opposite interconnecting end portions 19a of adjacent segments. The end portions as shown are generally elliptical but may be rounded or square or pointed or the like. Any configuration of end portions is acceptable so long as it provides an undulating pattern, as shown. When the flat form 10 is formed into an unexpanded tube as shown in Figure 2, the segments are cylindrical but the end portions 19 of adjacent segments remain in an opposed position relative to each other.

Interconnecting elements 20 extend from one end portion 19 of one segment 16 to another end portion 19 of another adjacent segment 16 but not to an oppositely positioned end portion 19 of an adjacent segment 16. There are at least three struts included between the points on each side of a segment 16 at which an interconnecting element 20 contacts an end portion 19. This results in the interconnecting elements 20 extending in an angular direction between segments around the periphery of the tubular stent. Interconnecting elements 20 are preferably of the same length but may vary from one segment to the other. Also, the diagonal direction may reverse from one segment to another extending upwardly in one case and downwardly in another, although all connecting elements between any pair of segments are substantially parallel. Figure 1, for example shows them extending downwardly, right to left. Upwardly would extend up left to right in this configuration.

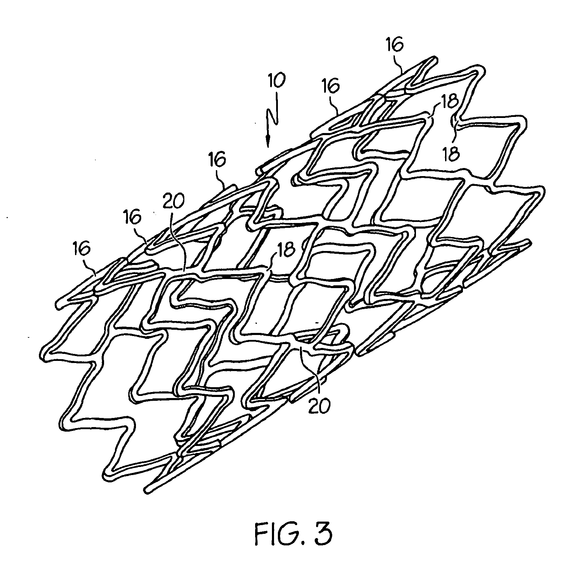

As a result of this angular extension of the interconnecting elements 20 between adjacent segments and loops, upon expansion of the stent as seen in Figure 3, the closest adjacent end portions 19 between segments 16 are displaced from each other and are no longer opposite each other so as to minimize the possibility of binding or overlapping between segments, i.e., pinching.

The number of interconnecting elements 20 may vary depending on circumstances in any particular instance. Three per segment are satisfactory for the configuration shown and at least three will be used typically.

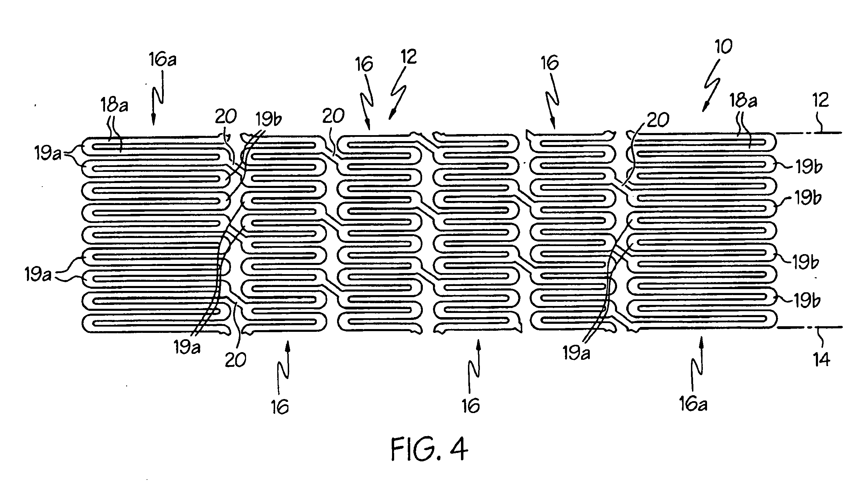

The alternate design shown in Figure 4 includes longer struts 18a in the two end segments 16a than in the intermediate segments 16. This allows the end segments (16a) to have less compression resistance than the intermediate segments (16), providing a more gradual transition from the native vessel to the support structure of the stent. Otherwise, the configuration is the same as that shown in Figure 1.

In some embodiments, the segments 16 can also be described as serpentine bands. The interconnecting elements 20 can also be described as connector struts. The end portions 19 can also be described as turns. End portions 19a can also be described as proximal peaks. End portions 19b can also be described as distal valleys.

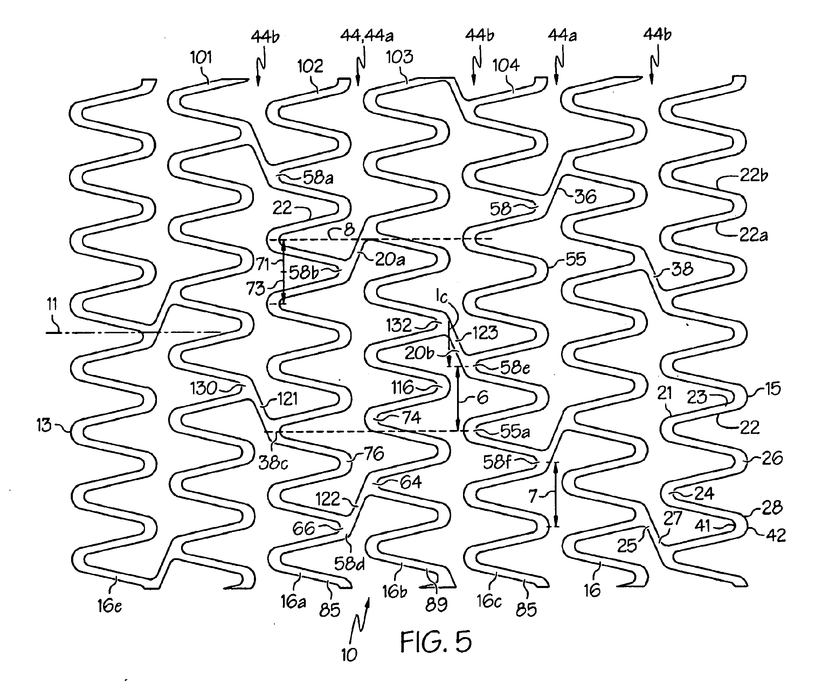

Figure 5 shows a flat pattern for an embodiment of a stent 10 having a proximal end 13, a distal end 15 and a plurality of serpentine bands 16. Each serpentine band 16 comprises a plurality of band struts 22 and a plurality of turns 28. The band struts 22 and the turns 28 alternate as the serpentine band 16 is traversed. Thus, each band strut 22 has a first end 21 connected to one turn 28 and a second end 23 connected to another turn 28. Each turn 28 connects between two band struts 22 that are adjacent to one another in a stent circumferential direction.

A band strut 22 is straight along its length as shown in Figure 5. In other examples not part of the invention, a band strut 22 can include curvature in one or more directions. A serpentine band 16 can further comprise band struts 22 that are shaped differently from one another. Other examples of possible configurations of band struts 22 are disclosed in US Patent Application Publication No. 2002/0095208 and US Patent Application No. 1 1/262692.

The turns 28 of a serpentine band 16 comprise alternating proximal peaks 24 and distal valleys 26. Each proximal peak 24 is generally convex with respect to the proximal end 13 and concave with respect to the distal end 15 of the stent 10. Each distal valley 26 is generally convex with respect to the distal end 15 and concave with respect to the proximal end 13 of the stent 10. Each turn 28 further comprises an inner side 41 and an outer side 43. Proximal peaks 24 are oriented with the outer side 43 closer to the proximal end 13 of the stent 10 than the inner side 41. Distal valleys 26 are oriented with the outer side 43 closer to the distal end 15 of the stent 10 than the inner side 41.

A stent 10 can have any suitable number of serpentine bands 16. In various embodiments, a serpentine band 16 can have any suitable number of band struts 22 and any suitable number of turns 28.

A serpentine band 16 can span any suitable distance along the length of the stent 10. In some embodiments, a stent 10 can comprise serpentine bands 16 that span different distances. One method for increasing a lengthwise span of a serpentine band 16 is to increase the length of the band struts 22.

In some embodiments, the proximal peaks 24 of a given serpentine band 16 are aligned around a common circumference of the stent 10, and the distal valleys 26 are similarly aligned around another common circumference of the stent 10. Each circumference can be oriented orthogonal to a longitudinal axis 11 of the stent 10. When turns 28 are aligned around a circumference, an extremity of the outer side 43 of each turn 28 can abut a common reference circumference. In some other embodiments, various peaks 24 can be offset from other peaks 24 within a given serpentine band 16, and various valleys 26 can be offset from other valleys 26 within the band 16.

Each band strut 22 comprises a width, which may be measured in a direction normal to the length of the strut 22. In some embodiments, all struts 22 within a given serpentine band 16 have the same width. In some embodiments, the width of various struts 22 within a serpentine band 16 can be different from one another. In some embodiments, the width of a strut 22 can change along the length of the strut 22. In some embodiments, the width of struts 22 of one serpentine band 16 can be different from the width of struts 22 of another serpentine band 16.

Each turn 28 has a width, which may be measured in a direction normal to the side of the turn 28 (e.g. normal to a tangent line). In some embodiments, the width of a turn 28 can be greater than the width of one or more struts 22 of the stent 10. In some embodiments, the width of a turn 28 can be less than the width of one or more struts 22 of the stent 10. In some embodiments, the width of a turn 28 varies from one end of the turn 28 to the other. For example, a turn 28 can connect to a strut 22 at one end having the same width as the strut 22. The width of the turn 28 increases, and in some embodiments reaches a maximum at a midpoint of the turn 28. The width of the turn 28 then decreases to the width of another strut 22, which may be connected to the second end of the turn 28.

Serpentine bands 16 that are adjacent to one another along the length of the stent 10 are connected by at least one connector strut 20. In some embodiments, a connector strut 20 spans between turns 28 of adjacent serpentine bands 20. For example, a first end 25 of a connector strut 20 can connect to a distal valley 26 of one serpentine band 16, and a second end 27 of the connector strut 20 can connect to a proximal peak 24 of an adjacent serpentine band 16.

Connector struts 16 can connect to any portion of a serpentine band 16.

In some embodiments, a connector strut 20 connects to a turn 28 as shown in Figure 5. In some examples not part of the invention, a connector strut 20 can connect to a band strut 22.

In some embodiments, a connector strut 20 is linear or straight along its length. In some embodiments, a connector strut 20 can include curvature along its length, and can further include multiple portions of curvature, for example a convex portion and a concave portion that may be connected at an inflection point.

Each connector strut 20 comprises a width, which may be measured in a direction normal to the length of the strut 20. In some embodiments, every connector strut 20 has the same width. In some other embodiments, a connector strut 20 can have a width that is different from another connector strut 20. In some embodiments, the width of a connector strut 20 can change along the length of the strut 20.

Some further examples of configurations that can be used for connector struts 16 are disclosed in US Patent Nos. 6261319 and 6478816, and US Published Patent Application No.20040243216.

Connector struts 20 comprise a first type of connector strut 36 and a second type of connector strut 38. A first connector strut 36 extends in a first direction. The first connector strut 36 is oriented at a first angle to a stent lengthwise axis 11. A second connector strut 38 extends in a second direction that is different than or non-parallel to the first direction. The second connector strut 38 can be oriented at a second angle to a stent lengthwise axis 11. In some embodiments, the first angle and the second angle can have the same magnitude but different orientations. For example, a first connector strut 36 can form a 70° angle with a stent lengthwise axis 11, while a second connector strut 38 can form a negative 70° angle with the stent lengthwise axis 11. In some embodiments, a first angle may comprise a mirror image of a second angle across a line parallel to the stent lengthwise axis 11. In some embodiments, first type of connector strut 36 can have a different shape than second type of connector strut 38.

In some embodiments, an area of the stent 10 located between two adjacent serpentine bands 16 can be considered a connector column 44. Each connector column 44 comprises a plurality of connector struts 20. Each connector strut 20 in a connector column 44 can be similar to one another. For example, each connector strut 20 in a first connector column 44a can comprise a first type connector strut 36. Each connector strut 20 in a second connector column 44b can comprise a second type of connector strut 38.

In some embodiments, first connector columns 44a and second connector columns 44b can alternate along the length of the stent 10. Thus, each interior serpentine band 16 can be positioned between a first connector column 44a and a second connector column 44b. Accordingly, connector struts 20 that connect to one side of a serpentine band 16 can comprise first connector struts 36, and connector struts 20 that connect to the other side of the serpentine band 16 can comprise second connector struts 38.

Turns 28 can comprise connected turns 58 or unconnected turns 55 depending upon whether the turn 28 connects to a connector strut 20. Similarly, proximal peaks 26 can comprise connected proximal peaks 64 or unconnected proximal peaks 74, and distal valleys 26 can comprise connected distal valleys 66 or unconnected distal valleys 76.

A serpentine band 16 can have more unconnected turns 55 than connected turns 58. In some embodiments, a serpentine band 16 has three unconnected turns 55 for each connected turn 58. The 3:1 ratio of unconnected turns 55 to connected turns 58 can also apply to the proximal peaks 24 and to the distal valleys 26.

In some embodiments, as a serpentine band 16 is traversed, there is a repeating pattern ofx number of unconnected turns 55 between one connected turn 58 and the next connected turn 58, and then y number of unconnected turns until the next connected turn 58, wherein y is greater than x. For example, referring to Figure 5, as a serpentine band 16a is traversed from a first connected turn 58a to a second connected turn 58b, there are two unconnected turns 55. Thus, x can equal two. As the serpentine band 16a is traversed from the second connected turn 58b to a third connected turn 58c, there are four unconnected turns 55. Thus, y can equal four. The pattern will then repeat, with x=2 unconnected turns 55 between the third connected turn 58c and a fourth connected turn 58d, etc. In some embodiments, y is a multiple of x, for example y=2x.

In some embodiments, starting from a connected turn 58, a serpentine band 16 can comprise three band struts 22 between the connected turn 58 and the next connected turn 58 in a first direction. The serpentine band 16 can further comprise five band struts 22 between the connected turn 58 and the next connected turn 58 in a second direction. For example, referring to Figure 5, a serpentine band 16a includes three band struts 22 between a connected turn 58b and the next connected turn 58a in a first circumferential direction 71. The serpentine band 16a also includes five band struts 22 between the connected turn 58b and the next connected turn 58c in a second circumferential direction 73.

In some embodiments, as a serpentine band 16 is traversed, there can be a repeating pattern of three band struts 22 between one connected turn 58 and the next connected turn 58, and then five band struts 22 until the next connected turn 58. For example, referring to Figure 5, as a serpentine band 16a is traversed from a first connected turn 58a to a second connected turn 58b, there are three band struts 22. As the serpentine band 16a is traversed from the second connected turn 58b to a third connected turn 58c, there are five band struts 22. The pattern will then repeat, with three band struts 22 between the third connected turn 58c and a fourth connected turn 58d, etc.

In some embodiments, an end serpentine band 16e that is located on the proximal end 13 or the distal end 15 of the stent 10 comprises seven unconnected turns 55 between two connected turns 58. The end serpentine band 16e can further comprise eight band struts 22 between two connected turns 58.

In some embodiments, the connector struts 20 of adjacent connector columns 44 are offset from one another in a stent circumferential direction. For example, one connector strut 20a is offset in a stent circumferential direction from another connector strut 20b located in an adjacent connector column 44. Thus, in some embodiments, a reference line 8 oriented parallel to the stent longitudinal axis 11 that intersects one connector strut 20a will not intersect the other connector strut 20b.

The band struts 22 of a serpentine band 16 can comprise alternating first band struts 22a and second band struts 22b. In some embodiments, each first band strut 22a is parallel to one another as shown in the flat pattern of Figure 5. Each second band strut 22b is parallel to one another and non-parallel to the first band struts 22a.

Serpentine bands 16 comprises a first type of serpentine band 85 and a second type of serpentine band 89. Each first type of serpentine band 85 is aligned with one another such that similar portions of each band 85 align along the length of the stent 10. Each second type of serpentine band 89 is aligned with one another such that similar portions of each band 89 align along the length of the stent 10. Each first type of serpentine band 85 is offset from each second type of serpentine band 89 such that similar portions of the different types of bands 85, 89 are not aligned along the length of the stent.

In some embodiments, the first type of serpentine band 85 and the second type of serpentine band 89 can alternate along the length of the stent 10. Thus, serpentine bands 16 that are located adjacent to one another along the length of the stent 10 can be offset from one another in a stent circumferential direction. Every other serpentine band 16 can be aligned with one another in a stent circumferential direction. For example, a stent 10 can comprise a first serpentine band 16a, a second serpentine band 16b and a third serpentine band 16c along its length. The first and third serpentine bands 16a, 16c comprise a first type of serpentine band 85, and the second serpentine band 16b comprises a second type of serpentine band 89. The first serpentine band 16a is offset from the second serpentine band 16b in a stent circumferential direction. Thus, a reference line 8 extending parallel to the stent longitudinal axis 11 will not intersect similar portions of the first serpentine band 16a and the second serpentine band 16b. As shown, the reference line 8 bisects a proximal peak 24 of the first serpentine band 16a but does not bisect a proximal peak 24 of the second serpentine band 16b. The second serpentine band 16b is similarly offset from the third serpentine band 16c. The first serpentine band 16a and the third serpentine band 16c are aligned with one another in a stent circumferential direction. Thus, the reference line 8 bisects a proximal peak 24 of both the first serpentine band 16a and the third serpentine band 16c.

One serpentine band 16 of a given type 85, 89 can have connected turns 58 that are aligned with unconnected turns 55 of another serpentine band 16 of the same type 85, 89 along the length of the stent 10. For example, the first serpentine band 16a of Figure 5 includes a connected turn 58c that is longitudinally aligned with an unconnected turn 55a of the third serpentine band 16c.

One serpentine band 16 of a given type 85, 89 can have connected turns 58 that are offset from connected turns 58 of the next adjacent serpentine band 16 of the same type 85, 89 by one proximal peak or one distal valley. For example, the first serpentine band 16a of Figure 5 includes a connected proximal peak 58c that is offset 6 from a connected proximal peak 58e of the third serpentine band 16c by one proximal peak 24. Similarly, the first serpentine band 16a includes a connected distal valley 58d that is offset from a connected distal valley 58f of the third serpentine band 16c by one distal valley 26. Thus, in some embodiments, the connector struts 20 of adjacent similar types of connector columns 44a, 44b are offset from one another in the stent circumferential direction by an amount equal to the spacing 6, 7 between adjacent proximal peaks 24 or between adjacent distal valleys 26.

Referring to Figures 1 and 5, in some embodiments, a stent comprises at least a first serpentine band 101, a second serpentine band 102, a third serpentine band 103 and a fourth serpentine band 104. Each serpentine band 101-104 comprises connected proximal peaks 64, unconnected proximal peaks 74, connected distal valleys 66 and unconnected distal valleys 76. Each serpentine band 101-104 includes at least two unconnected proximal peaks 74 for each connected proximal peak 64, and at least two unconnected distal valleys 76 for each connected distal valley 66.

A first connector strut 121 connects between a first connected distal valley 130, located on the first serpentine band 101, and a connected proximal peak 64 of the second serpentine band 102. A second connector strut 122 connects between the second serpentine band 102 and the third serpentine band 103. A third connector strut 123 connects between a second connected distal valley 132, located on the third serpentine band 103, and a connected proximal peak 64 of the fourth serpentine band 104.

The first connected distal valley 130 is circumferentially aligned with a first unconnected distal valley 116 of the third serpentine band 103. The first unconnected distal valley 116 is directly adjacent in a circumferential direction to the second connected distal valley 132.

Each connected distal valley 66 of the first serpentine band 101 is circumferentially aligned with an unconnected distal valley 76 of the third serpentine band 103. Further, each unconnected distal valley 76 of the third serpentine band 103 that is circumferentially aligned with a connected distal valley 66 of the first, serpentine band 101 is offset from a connected distal valley 66 of the third serpentine band 103 in a circumferential direction by one distal valley (e.g. spacing 7 as shown on Figure 5).

The third connector strut 123 is oriented at a non-zero angle to the stent longitudinal axis 11 and thus comprises a circumferential length component *l_{c}* oriented in a stent circumferential direction. The circumferential length component *l_{c}* extends from the second connected distal valley 132 in a circumferential direction toward the first unconnected distal valley 116. Thus, in some embodiments, connector struts 20 that connect to connected distal valleys 66 of the third serpentine band 103 extend at an angle to the stent longitudinal axis 11, wherein the angle is oriented in the direction of an adjacent unconnected distal valley 76 (e.g. distal valley 116) that is circumferentially aligned with a connected distal valley 66 (e.g. distal valley 130) of the first serpentine band 101.

The second serpentine band 102 comprises three band struts 22 between the first connector strut 121 and the second connector strut 122. Thus, there are three band struts 22 located between the connected distal valley 66 that connects to the first connector strut 121 and the connected proximal peak 64 that connects to the second connector strut 122.

Each connected distal valley 66 of the second serpentine band 102 is circumferentially aligned with an unconnected distal valley 76 of the fourth serpentine band 104. Further, each unconnected distal valley 76 of the fourth serpentine band 104 that is circumferentially aligned with a connected distal valley 66 of the second serpentine band 102 is offset from a connected distal valley 66 of the fourth serpentine band 104 in a circumferential direction by one distal valley (e.g. spacing 7 as shown on Figure 5).

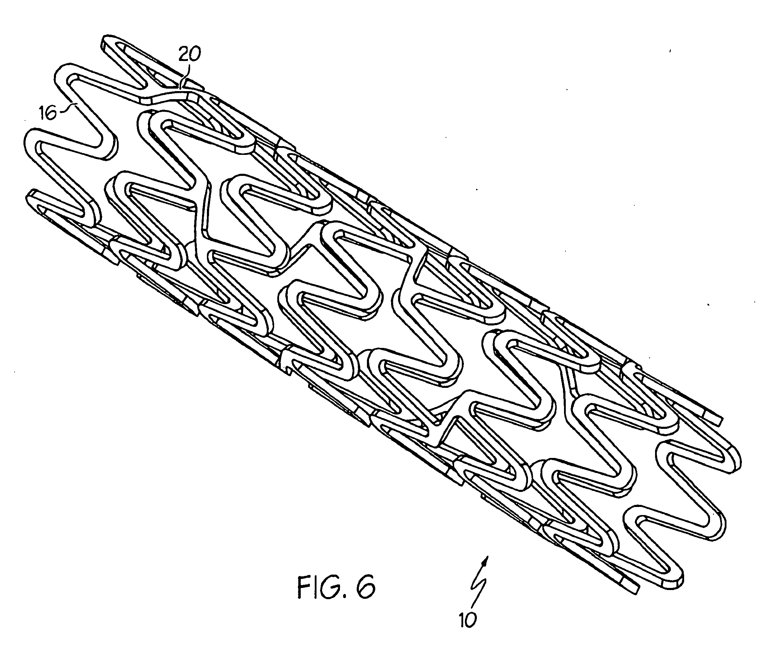

Figure 6 shows a three-dimensional substantially cylindrical stent 10 according to the flat pattern shown in Figure 5. The stent 10 is shown at a nominal state of expansion and could be further reduced in diameter, for example being crimped onto a delivery catheter, or could be further expanded.

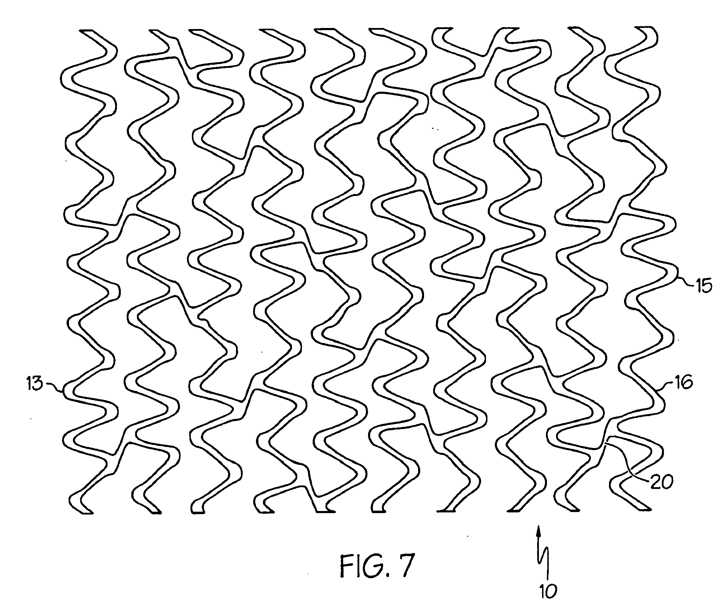

Figure 7 shows an example of a stent 10 in a state of expansion that is greater than that of Figure 5.

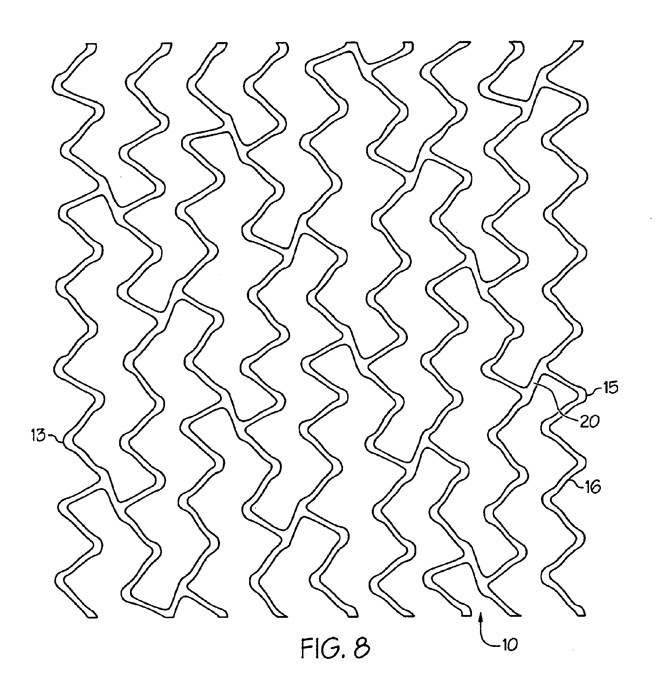

Figure 8 shows an example of a stent 10 in a state of expansion that is greater than that of Figure 7.

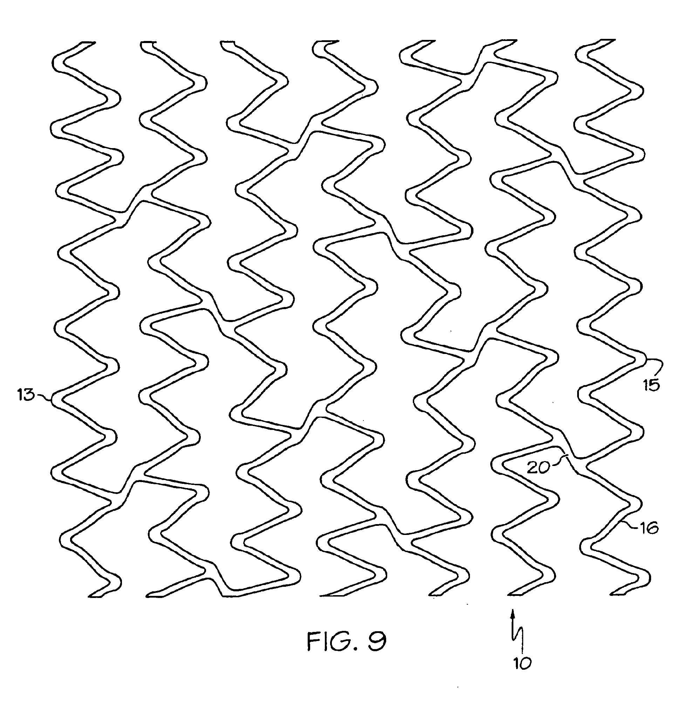

Figure 9 shows an example of a stent 10 in a state of expansion that is greater than that of Figure 8.

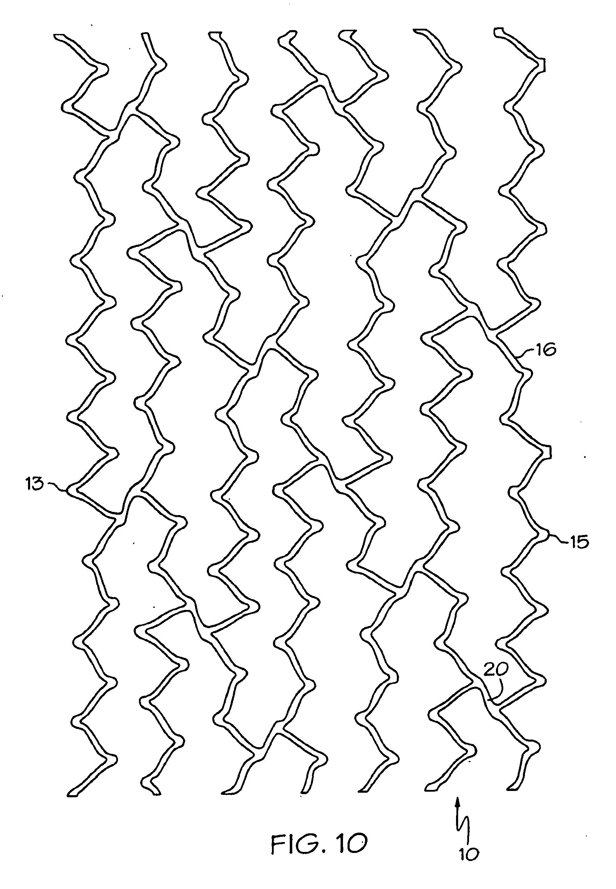

Figure 10 shows an example of a stent 10 in a state of expansion that is greater than that of Figure 9. The amount of expansion depicted can be described as a state of overexpansion. Generally, a stent 10 that is actually used in a bodily vessel will be subject to less expansion than the amount shown in Figure 10. However, the stent 10 pattern shown is capable of providing vessel support even in a substantially overexpanded state.

The stent as disclosed in the appended claims may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, co-polymers of these materials as well as composites and combinations thereof and combinations of other biodegradable polymers. Other polymers that may be used include polyester and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The stent as disclosed in the appended claims, may be made of shape memory materials such as superelastic Nitinol or spring steel, or may be made of materials which are plastically deformable. In the case of shape memory materials, the stent may be provided with a memorized shape and then deformed to a reduced diameter shape. The stent may restore itself to its memorized shape upon being heated to a transition temperature and having any restraints removed therefrom.

The stents as disclosed in the appended claims, may be created by methods including cutting or etching a design from a tubular stock, from a flat sheet which is cut or etched and which is subsequently rolled or from one or more interwoven wires or braids. Any other suitable technique which is known in the art or which is subsequently developed may also be used to manufacture the inventive stents disclosed herein.

In some embodiments the stent, the delivery system or other portion of the assembly may include one or more areas, bands, coatings, members, etc. that is (are) detectable by imaging modalities such as X-Ray, MRI, ultrasound, etc. In some embodiments at least a portion of the stent and/or adjacent assembly is at least partially radiopaque.

In some embodiments the at least a portion of the stent is configured to include one or more mechanisms for the delivery of a therapeutic agent. Often the agent will be in the form of a coating or other layer (or layers) of material placed on a surface region of the stent, which is adapted to be released at the site of the stent's implantation or areas adjacent thereto.

A therapeutic agent may be a drug or other pharmaceutical product such as non-genetic agents, genetic agents, cellular material, etc. Some examples of suitable non-genetic therapeutic agents include but are not limited to: anti-thrombogenic agents such as heparin, heparin derivatives, vascular cell growth promoters, growth factor inhibitors, Paclitaxel, etc. Some other examples of therapeutic agents include everolimus and sirolimus, their analogs and conjugates. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

The above disclosure is intended to be illustrative and not exhaustive. This description will suggest many variations and alternatives to one of ordinary skill in this art. The various elements shown in the individual figures and described above may be combined or modified for combination as desired. All these alternatives and variations are intended to be included within the scope of the claims where the term "comprising" means "including, but not limited to".

## Claims

1. A stent (10) comprising
• a plurality of serpentine bands (16) and a plurality of connector columns (44), each serpentine band comprising a plurality of alternating straight band struts (22) and turns,
• adjacent serpentine bands (16) connected across a connector column (44) by a plurality of connector struts (20),
• each connector strut (20) connected at one end to a turn of one serpentine band (16) and connected at the other end to a turn of another serpentine band (16),
• the turns of a serpentine band comprising connected turns (58) that connect to a connector strut and unconnected turns (55) that do not connect to a connector strut (20),
• at least one of the serpentine bands (16) comprising a repeating pattern of three band struts (22) and then five band struts (22) extending between connected turns (58) as the serpentine band (16) is traversed,
• the connector columns (44) comprising first connector columns (44a) and second connector columns (44b), connector struts (20a) of the first connector columns (44a) being parallel to one another, connector struts (20b) of the second connector columns (44b) being nonparallel to the connector struts (20) of the first connector columns (44a),
**characterized in that**
• the serpentine bands (16) comprise first serpentine bands (85) and second serpentine bands (89), turns of the first serpentine bands (85) aligned with one another in a stent longitudinal direction, turns of the first serpentine bands (85) offset from turns of the second serpentine bands (89) in a stent longitudinal direction and

2. The stent of claim 1, wherein a plurality of the serpentine bands (16) comprise a repeating pattern of three band struts (22) and then five band struts extending between connected turns.

3. The stent of claim 2, comprising at least one serpentine band (16) having eight band struts extending between connected turns (58).

4. The stent of claim 3, wherein first serpentine bands (16) and second serpentine bands (16) alternate along the length of the stent (10).

5. The stent of claim 4, wherein a connected turn (58) of a first serpentine band is aligned with an unconnected turn (55) of an adjacent first serpentine band (16) in a stent longitudinal direction.

6. The stent of claim 5, wherein first connector columns (44) and second connector columns (44) alternate along the length of the stent.

7. The stent of claim 1, wherein a maximum width of a band strut (22) is less than a maximum width of a turn.

8. The stent of claim 1, wherein the turns of a serpentine band comprise alternating proximal peaks and distal valleys, the proximal peaks of a serpentine band (16) being aligned about a common circumference of the stent (10).

9. The stent of claim 8, wherein a given serpentine band comprises three band struts (22) extending between a first connected proximal peak (64) that connects to a connector strut (20) and a first connected distal valley (130) that connects to a connector strut (20), and further comprises five band struts (22) extending between the first connected distal valley (130) and a second connected proximal peak (64).

10. The stent of claim 9, the given serpentine band (16) further comprising three band struts (22) extending between the second connected proximal peak (64) and a second connected distal valley (132), and five band struts (22) extending between the second connected distal valley (132) and the first connected proximal peak (64).

11. The stent of claim 1, a given serpentine band comprising two unconnected turns (55) between a first connected turn (58) and a second connected turn (58) and four unconnected turns (55) between the second connected turn (58) and a third connected turn (58) as the serpentine band (16) is traversed.

12. The stent of claim 11, wherein the given serpentine band (16) further comprises two unconnected turns (55) between the third connected turn (58) and a fourth connected turn (58) and four unconnected turns (55) between the fourth connected turn (58) and the first connected turn (58) as the serpentine band (16) is traversed.

13. The stent of claim 12, wherein connector struts (20) that connect to the first connected turn (58) and the third connected turn (58) are parallel to one another and nonparallel to connector struts (20) that connect to the second connected turn (58) and the fourth connected turn (58).

## Patentansprüche

1. Stent (10) umfassend
• eine Vielzahl von serpentinenförmigen Bändern (16) und eine Vielzahl von Verbindersäulen (44), wobei jedes serpentinenförmige Band eine Vielzahl von geraden Bandstreben (22) und Kehren umfasst, die sich abwechseln,
• aneinander angrenzende serpentinenförmige Bänder (16), die quer über eine Verbindersäule (44) hinweg durch eine Vielzahl von Verbinderstreben (20) verbunden sind,
• wobei jede Verbinderstrebe (20) an einem Ende mit einer Kehre eines serpentinenförmigen Bandes (16) verbunden ist und an dem anderen Ende mit einer Kehre eines anderen serpentinenförmigen Bandes (16) verbunden ist,
• wobei die Kehren eines serpentinenförmigen Bandes verbundene Kehren (58) umfassen, welche zu einer Verbinderstrebe verbinden, sowie unverbundene Kehren (55), die nicht zu einer Verbinderstrebe (20) verbinden,
• wobei mindestes eines der serpentinenförmigen Bänder (16) ein sich wiederholendes Muster aus drei Bandstreben (22) und dann fünf sich zwischen verbundenen Kehren (58) erstreckenden Bandstreben (22) umfasst, wenn das serpentinenförmige Band (16) durchquert wird,
• wobei die Verbindersäulen (44) erste Verbindersäulen (44a) und zweite Verbindersäulen (44b) umfassen, Verbinderstreben (20a) der ersten Verbindersäulen (44a) parallel zueinander sind und Verbinderstreben (20b) der zweiten Verbindersäulen (44b) nicht parallel zu den Verbinderstreben (20) der ersten Verbindersäulen (44a) sind,
**dadurch gekennzeichnet dass**
• die serpentinenförmigen Bänder (16) erste serpentinenförmige Bänder (85) und zweite serpentinenförmige Bänder (89) umfassen, Kehren der ersten serpentinenförmigen Bänder (85) in der Längsrichtung des Stents zueinander ausgerichtet sind und Kehren der ersten serpentinenförmigen Bänder (85) in der Längsrichtung des Stents gegenüber Kehren der zweiten serpentinenförmigen Bänder (89) versetzt sind.

2. Stent gemäß Anspruch 1, wobei eine Vielzahl der serpentinenförmigen Bänder (16) ein sich wiederholendes Muster aus drei Bandstreben (22) und dann fünf sich zwischen verbundenen Kehren erstreckenden Bandstreben umfassen.

3. Stent gemäß Anspruch 2, umfassend mindestens ein serpentinenförmiges Band (16) mit acht sich zwischen verbundenen Kehren (58) erstreckenden Bandstreben.

4. Stent gemäß Anspruch 3, wobei erste serpentinenförmige Bänder (16) und zweite serpentinenförmige Bänder (16) sich entlang der Länge des Stents (10) abwechseln.

5. Stent gemäß Anspruch 4, wobei eine verbundene Kehre (58) eines ersten serpentinenförmigen Bandes mit einer unverbundenen Kehre (55) eines angrenzenden ersten serpentinenförmigen Bandes (16) in der Längsrichtung des Stents fluchtet.

6. Stent gemäß Anspruch 5, wobei erste Verbindersäulen (44) und zweite Verbindersäulen (44) sich entlang der Länge des Stents abwechseln.

7. Stent gemäß Anspruch 1, wobei die maximale Breite einer Bandstrebe (22) geringer ist als die maximale Breite einer Kehre.

8. Stent gemäß Anspruch 1, wobei die Kehren eines serpentinenförmigen Bandes sich abwechselnde proximale Gipfel und distale Täler umfassen, wobei die proximalen Gipfel eines serpentinenförmigen Bandes (16) über einem gemeinsamen Umfang des Stents (10) ausgerichtet sind.

9. Stent gemäß Anspruch 8, wobei ein gegebenes serpentinenförmiges Band drei Bandstreben (22) umfasst, die sich zwischen einem ersten verbundenen proximalen Gipfel (64), welcher zu einer Verbinderstrebe (20) verbindet, und einem ersten verbundenen distalen Tal (130) erstrecken, welches zu einer Verbinderstrebe (20) verbindet, und es ferner fünf sich zwischen dem ersten verbundenen distalen Tal (130) und einem zweiten verbundenen proximalen Gipfel (64) erstreckende Bandstreben (22) umfasst.

10. Stent gemäß Anspruch 9, wobei das gegebene serpentinenförmige Band (16) ferner drei Bandstreben (22) umfasst, die sich zwischen dem zweiten verbundenen proximalen Gipfel (64) und einem zweiten verbundenen distalen Tal (132) erstrecken, sowie fünf Bandstreben (22), die sich zwischen dem zweiten verbundenen distalen Tal (132) und dem ersten verbundenen proximalen Gipfel (64) erstrecken.

11. Stent gemäß Anspruch 1, wobei ein gegebenes serpentinenförmiges Band zwei unverbundene Kehren (55) zwischen einer ersten verbundenen Kehre (58) und einer zweiten verbundenen Kehre (58) sowie vier unverbundene Kehren (55) zwischen der zweiten verbundenen Kehre (58) und einer dritten verbundenen Kehre (58) umfasst, wenn das serpentinenförmige Band (16) durchquert wird.

12. Stent gemäß Anspruch 11, wobei das gegebene serpentinenförmige Band (16) ferner zwei unverbundene Kehren (55) zwischen der dritten verbundenen Kehre (58) und einer vierten verbundenen Kehre (58) umfasst, sowie vier unverbundene Kehren (55) zwischen der vierten verbundenen Kehre (58) und der ersten verbundenen Kehre (58), wenn das serpentinenförmige Band (16) durchquert wird.

13. Stent gemäß Anspruch 12, wobei Verbinderstreben (20), welche zu der ersten verbundenen Kehre (58) und der dritten verbundenen Kehre (58) verbinden, parallel zueinander und nicht-parallel zu Verbinderstreben (20) sind, welche zu der zweiten verbundenen Kehre (58) und der vierten verbundenen Kehre (58) verbinden.

## Revendications

1. Stent (10) comportant
• une pluralité de bandes en serpentin (16) et une pluralité de colonnes de raccordement (44), chaque bande en serpentin comportant une pluralité de plaquettes de bande droites (22) et de tours alternants,
• des bandes en serpentin (16) adjacentes, raccordés à travers une colonne de raccordement (44) par une pluralité de plaquettes de raccordement (20),
• chaque plaquette de raccordement (20) étant raccordée dans une extrémité à un tour d'une bande en serpentin (16), et dans l'autre extrémité à un tour d'une autre bande en serpentin (16),
• les tours d'une bande en serpentin comprenant des tours raccordés (58), qui raccordent à une plaquette de raccordement, et des tours non raccordés (55), qui ne raccordent pas à une plaquette de raccordement (20),
• au moins une des bandes en serpentin (16) comportant un motif répété de trois plaquettes de bande (22) et puis de cinq plaquettes de bande (22) qui s'étendent entre des tour raccordés (58) à mesure que la bande en serpentin (16) est traversée,
• les colonnes de raccordement (44) comportant premières colonnes de raccordement (44a) et deuxièmes colonnes de raccordement (44b), les plaquettes de raccordement (20a) des premières colonnes de raccordement (44a) étant parallèles l'une à l'autre et les plaquettes de raccordement (20b) des deuxièmes colonnes de raccordement (44b) n'étant pas parallèles aux plaquettes de raccordement (20) des premières colonnes de raccordement (44a),
**caractérisé en ce que**
• les bandes en serpentin (16) comportent premières bandes en serpentin (85) et deuxièmes bandes en serpentin (89), tours des premières bandes en serpentin (85) sont alignés dans la direction de longueur du Stent et tours des première bandes en serpentin (85) sont décalés des tours des deuxièmes bandes en serpentin (89) dans la direction de longueur du Stent.

2. Stent selon la revendication 1, dans lequel une pluralité des bandes en serpentin (16) comportent un motif répété de trois plaquettes de bande (22) et puis de cinq plaquettes de bande qui s'étendent entre des tours raccordés.

3. Stent selon la revendication 2, comportant au moins une bande en serpentin (16) avec huit plaquettes de bande qui s'étendent entre des tours raccordés (58).

4. Stent selon la revendication 3, dans lequel premières bandes en serpentin (16) et deuxièmes bandes en serpentin (16) alternent le long de la longueur du Stent (10).

5. Stent selon la revendication 4, dans lequel un tour raccordé (58) d'une première bande en serpentin est aligné dans la direction de longueur du Stent à un tour non raccordé (55) d'une première bande en serpentin (16) adjacente.

6. Stent selon la revendication 5, dans lequel premières colonnes de raccordement (44) et deuxièmes colonnes de raccordement (44) alternent le long de la longueur du Stent.

7. Stent selon la revendication 1, dans lequel la largueur maximale d'une plaquette de bande (22) est inférieure à la largueur maximale d'un tour.

8. Stent selon la revendication 1, dans lequel les tours d'une bande en serpentin comportent des sommets proximaux et des vallées distales alternants, les sommets proximaux d'une bande en serpentin (16) étant alignés autour d'une circonférence commune du Stent (10).

9. Stent selon la revendication 8, dans lequel une bande en serpentin donnée comporte trois plaquettes de bande (22) qui s'étendent entre un premier sommet raccordé proximal (64) qui raccorde à une plaquette de raccordement (20), et une première vallée raccordée distale (130) qui raccorde à une plaquette de raccordement (20), et comporte en outre cinq plaquettes de bande (22) qui s'étendent entre la première vallée raccordée distale (130) et un deuxième sommet raccordé proximal (64).

10. Stent selon la revendication 9, dans lequel la bande en serpentin (16) donnée comporte en outre trois plaquettes de bande (22) qui s'étendent entre le deuxième sommet raccordé proximal (64) et une deuxième vallée raccordée distale (132), et cinq plaquettes de bande (22), qui s'étendent entre la deuxième vallée racordée distale (132) et le premier sommet raccordé proximal (64).

11. Stent selon la revendication 1, dans lequel une bande en serpentin donnée comporte en outre deux tours non raccordés (55) entre un premier tour raccordé (58) et un deuxième tour raccordé (58), et quatre tours non raccordés (55) entre le deuxième tour raccordé (58) et un troisième tour raccordé (58) à mesure que la bande en serpentin (16) est traversée.

12. Stent selon la revendication 11, dans lequel la bande en serpentin (16) donnée comporte en outre deux tours non raccordés (55) entre le troisième tour raccordé (58) et un quatrième tour raccordé (58), et quatre tours non raccordés (55) entre le quatrième tour raccordé (58) et le premier tour raccordé (58) à mesure que la bande en serpentin (16) est traversée.

13. Stent selon la revendication 12, dans lequel des plaquettes de raccordement (20) qui raccordent au premier tour raccordé (58) et au troisième tour raccordé (58) sont parallèles l'une à l'autre, et ne pas parallèles à des plaquettes de raccordement (20) qui raccordent au deuxième tour raccordé (58) et au quatrième tour raccordé (58).
